# EUROPEAN PATENT SPECIFICATION

(11) **EP 1 098 678 B1**
(45) Date of publication and mention of the grant of the patent: **03.07.2002**
(21) Application number: 99915912.2
(22) Date of filing: 09.04.1999
(51) Int. Cl.: A61N 5/06

(54) **LASER TREATMENT COOLING HEAD**
KÜHLKOPF FÜR EINEN LASERBEHANDLUNGSAPPARAT
TETE DE REFROIDISSEMENT POUR TRAITEMENT LASER

(30) Priority: 09.04.1998 GB 9807794
(43) Date of publication of application: 16.05.2001
(73) Proprietor: COOLANALGESIA LIMITED, Cambridge CB2 1PH (GB)
(72) Inventor: Whitcroft, Ian Andrew, Huron, SD 57350 (US); McMahon, Richard Anthony, Cambridge CB5 8HL (GB)
(74) Representative: Brunner, Michael John
(86) International application number: GB9901107
(87) International publication number: WO9952595

(56) References cited:
- EP-A- 0 783 904
- US-A- 5 085 657
- US-A- 5 344 418
- US-A- 5 486 172

## Description

The present invention relates generally to means of cooling the surface of skin during laser treatment (resurfacing), the aim being to reduce the sensitivity to pain, reduce the need for anaesthetic or both. More particularly, the present invention relates to a laser treatment cooling head and, particularly, to a cooling head which can be used with a CO₂ or other laser employed for laser resurfacing of a patient's skin.

Such a device is for example known from US-A-5 486 172.

The advent of easily usable laser equipment has led to a great increase in this kind of treatment, but the problem of the control of pain, which can be severe as the process is essentially one of burning away skin, remains an obstacle to the wider use of the technique. In particular, according to clinical judgment and patient tolerance of pain, a general or local anaesthetic may be necessary. The use of a general anaesthetic enables the clinician to proceed rapidly with the treatment without constant checking of the comfort of the patient, but carries the severe drawbacks of the well known trauma of general anaesthetics, including the risk and the need for a recovery time before the patient resumes normal life, the need for an anaesthetist and the time for preparation and recovery. The latter two, in particular, make the cost of the treatment very high. In contrast, use of a local anaesthetic can allow a dermatologist to administer the anaesthetic, but again there are drawbacks. Pain control over certain areas of the body, particularly the face, requires multiple injection sites which is traumatic for the patient and the need for top-up doses during treatment is also unpleasant for the patient, also slowing down the treatment. The total dose of local anaesthetic is also limited which can therefore limit a particular treatment session, thus requiring further sessions and associated increased cost.

It has been proposed to use localised cooling to overcome the problems of anaesthesia and. In particular, it has been proposed to utilise a flow of coolant through a laser-transparent glass chamber which can be held against the skin while the laser treatment is carried out. However, this is only possible with visible light lasers, not with C02 or Erbium YAG (yitrium aluminium garnet) lasers, as existing cooling devices use coolants and windows that are not lucent to the wavelengths of C02 or Erbium YAG radiation, i.e. current cooling devices using water would absorb the energy at these wavelengths so skin treatment would be impossible.

According to the present invention, there is provided a cooling head for a skin treatment laser, the cooling head comprising
a body having
a mount at one end for attachment to a laser head to secure the body relative to the head in a fixed position;
a cooling surface at the other end for application to a patient's skin;
a coolant chamber disposed between the mount and the cooling surface, the chamber having
   wall portions transparent to CO₂, Erbium YAG or other non-visible laser light to allow a laser beam to be passed from the mount to the patient's skin through the cooling surface, and
   a coolant inlet and outlet provided in the body to enable the supply of coolant to and the removal of coolant from the body in use in order to cool the cooling surface;
an extraction port for enabling removal of debris from the treatment area and for connection to a vacuum source; and
a window in the body optically transparent in the visible light range to allow the laser beam at the cooling surface to be viewed through the body by a surgeon using the laser.

Preferably, the transparent wall portions comprise a pair of aligned windows on opposite sides of the coolant chamber, one adjacent the mount and the other in or adjacent to the cooling surface.

In a first embodiment, the extraction port may be connected to an aperture in the cooling surface adjacent the wall portion in the cooling surface through which the laser light impinges on the patient's skin, but, alternatively, a flexible skirt may be provided around the cooling surface to form an extraction chamber of variable volume and to which the extraction port, together with an air inlet port, may be connected for debris removal. During treatment, the cooling head is pushed onto the skin so that the cooling surface is in contact with the skin and thereby cools the skin. The head is lifted off at times during treatment and at the end of treatment, when the skirt provides some sealing to help with debris removal. The skirt may be very compliant neoprene.

The laser light transparent wall portion in the cooling surface may also be optically transparent in the visible spectrum to provide for viewing of the skin surface by the surgeon or else a separate transparent window may be provided. A further visible light transparent window is preferably disposed in an angled side wall of the body facing towards the aperture in the cooling surface. One or more of the transparent windows may be in the form of a lens to magnify the treatment area.

At least the cooling surface of the body may be formed of metal in order to enable the surface to be efficiently cooled by the flow of coolant through coolant chamber in the body so as to provide cooling to the patient's skin by the application of the cooled surface to the skin.

Using a metal body enables a relatively large mass of cold material to be provided as, in effect, a store of refrigerated material, ensuring that cooling around the treatment area can be maintained with a high degree of certainty.

A CO₂ laser would be incapable of use with the prior art systems mentioned above and therefore the present invention enables the direct application of CO₂ or YAG laser energy to the skin, whilst ensuring that both satisfactory cooling and required visibility for the surgeon are maintained.

Preferably the mount comprises an annular counter-bore at the open end of the bore and has fastenings for securing a laser head in a defined position within the counter-bore.

The surface applied to the patient's skin may be substantially flat or significantly curved, depending on the precise application, ie the particular area of the skin to be treated. For example, when treating significantly concave areas of the skin, such as adjacent the nose, a convex surface in necessary in order that as much of the surface as possible can engage the skin to provide an effective seal with the skin.

Preferably, the coolant passage comprises inlet and outlet ports and an internal chamber formed within the body and opening to the patient's skin through the surface. The coolant may be a low temperature gas such as nitrogen which may be removed through the extraction port together with debris, by a suitable vacuum pump or the like.

A light may be provided within the chamber to illuminate the treatment area.

One examples of a cooling head according to the present invention will now be described with reference to the accompanying drawings in which :

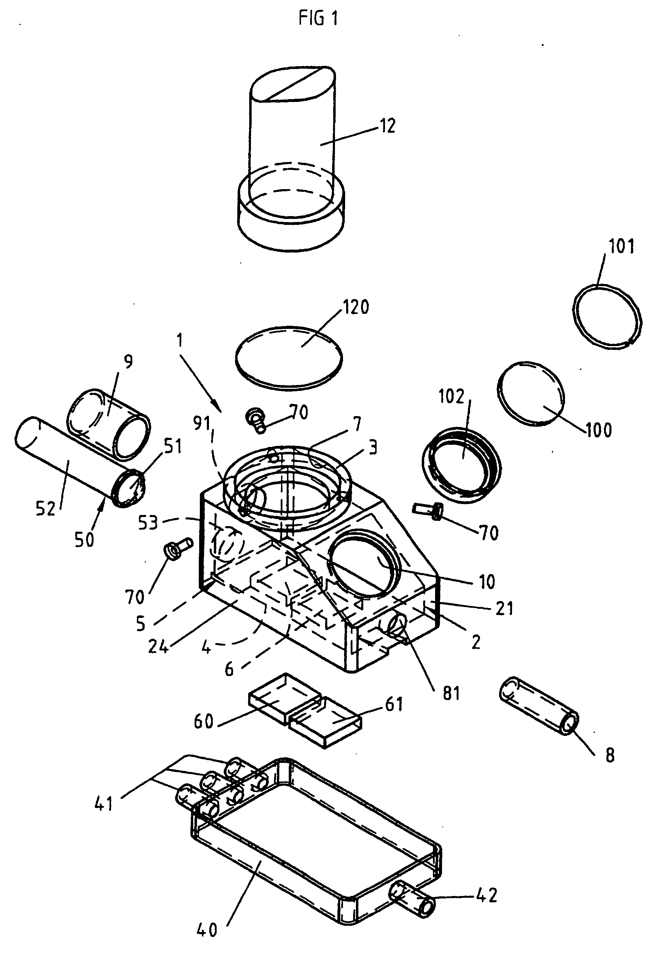

Figure 1 is an exploded view; and

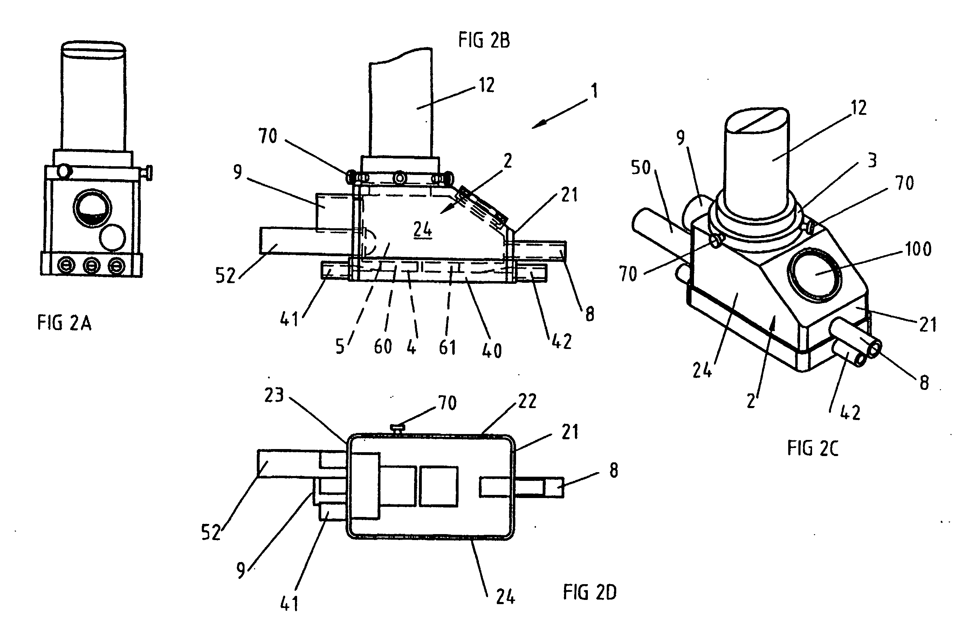

Figures 2A-D show the assembled head in a number of views from different positions.

The cooling head 1 shown in the drawings comprises a generally box-like die-cast or injection moulded body 2 having an annular mounting lip 3 on its top, at the other end a cooling surface 4, and sidewalls 21-24, with a pair of apertures 6 formed in the end face 4, into which a pair of windows 60,61 are provided. A first window 60 is transparent to CO₂ or Erbium YAG laser light to allow a laser beam to be passed therethrough and a second window 61 is transparent in the visible light range. Within the body 2 is a chamber 5 which extends from the mount 3 connecting with it through a bore or passage 7.

The annular lip 3 provides for accurate and secure location of the lower end of a laser housing 12, three locking screws 70 serving to locate the lower end of the tubular housing 12 for the laser within the bore or passage 7. A further window 120, which is transparent to CO₂ or Erbium YAG laser light, allows the laser beam to be passed therethrough to the window 60 and hence to the patient's skin.

A pair of tubular coolant ports 8,9 are fixed to the sidewalls 21,23 respectively at corresponding apertures 81,91 to provide for the outflow and inflow respectively of coolant from and to the chamber 5.

An inclined bore sidewall 25 has a circular aperture 10 into the chamber 5, which thus provides for a viewing window. Within the aperture 10 is fitted a lens 100, held in place by a circlip 101 and sealed to the side of the aperture with an O-ring synthetic rubber seal 102. The aperture 10 is directed towards the aperture 6 which contains the window 61 to enable the surgeon to view, with slight magnification, the area of skin being treated whilst treatment is actually taking place.

A neoprene rubber skirt 40 is provided around the cooling surface 4 and has inlets 41 and an outlet 42 through which air or another suitable fluid are drawn by a pump such as a vacuum pump, in order to remove debris from the patient's skin during treatment, the surgeon lifting the cooling surface 4 from the skin temporarily, allowing the fluid to entrain the debris for removal.

In use, the body is pre-cooled my means of the flow of refrigerant through the chamber 5, whilst attached to the laser head housing 12, and is then applied to the skin of the patient, the flexible skirt 40 allowing the cooling surface 4 to engage the skin, to cool the area of treatment, debris from the skin being removed through the outlet port 42. Coolant is provided to the chamber through the port 9 and spent coolant, which may be filtered and recooled for re-circulation through the chamber 5, is removed from the port 8.

To increase visibility of the treatment area, a light 50 can be provided inside the chamber 5, by means of a suitable LED 51 mounted on a tube 52 extending into the chamber through an aperture 53 in the sidewall 23.

The head may be anatomically shaped to suit particular areas. The contact head may be articulated to allow the head to move smoothly over anatomical obstacles and contours (eg from cheek to nose) more easily whilst maintaining intimate skin contact. The head may be arranged to rotate, for example, between specific indexed orientations, to enable the direction of traverse to be changed without having to rotate the whole body.

Furthermore, the contact head may be made as a disposable component.

## Claims

1. A cooling head (1) for a skin treatment laser, the cooling head comprising
a body (2) having
a mount (3) at one end for attachment to a laser head to secure the body relative to the head in a fixed position;
a cooling surface (4) at the other end for application to a patient's skin;
a coolant chamber (5) disposed between the mount and the cooling surface, the chamber having
wall portions (60, 61) transparent to CO₂, Erbium YAG or other non-visible laser light to allow a laser beam to be passed from the mount to the patient's skin through the cooling surface, and
a coolant inlet (9) and outlet (8) provided in the body to enable the supply of coolant to and the removal of coolant from the body in use in order to cool the cooling surface;
an extraction port (42) for enabling removal of debris from the treatment area and for connection to a vacuum source; and
a window (100) in the body optically transparent in the visible light range to allow the laser beam at the cooling surface to be viewed through the body by a surgeon using the laser.

2. A cooling head according to claim 1, wherein the transparent wall portions comprise a pair of aligned windows on opposite sides of the coolant chamber, one adjacent the mount and the other in or adjacent to the cooling surface.

3. A cooling head according to claim 1 or claim 2, wherein the extraction port is connected to an aperture in the cooling surface adjacent the wall portion in the cooling surface through which the laser light impinges on the patient's skin.

4. A cooling head according to claim 1 or claim 2, wherein, a flexible skirt is provided around the cooling surface to form an extraction chamber of variable volume and to which the extraction port, together with an air inlet port, may be connected for debris removal.

5. A cooling head according to any of claims 1 to 4, wherein the laser light transparent wall portion in the cooling surface is optically transparent in the visible spectrum to provide for viewing of the skin surface by the surgeon.

6. A cooling head according to any of claims 1 to 4, wherein a separate transparent window may be provided in the cooling surface to provide for viewing of the skin surface by the surgeon.

7. A cooling head according to any of claims 1 to 6, wherein a visible light transparent window is disposed in an angled side wall of the body facing towards the aperture in the cooling surface.

8. A cooling head according to any of claims 5 to 7, wherein one or more of the transparent windows is in the form of a lens to magnify the treatment area.

9. A cooling head according to any of claims 1 to 8, wherein at least the cooling surface of the body is formed of metal in order to enable the surface to be efficiently cooled by the flow of coolant through coolant chamber in the body so as to provide cooling to the patient's skin by the application of the cooled surface to the skin.

10. A cooling head according to any of claims 1 to 9, wherein the mount comprises an annular counter-bore at the open end of the bore and has fastenings for securing a laser head in a defined position within the counter-bore.

11. A cooling head according to any of claims 1 to 10, wherein the coolant passage comprises inlet and outlet ports and an internal chamber formed within the body and opening to the patient's skin through the surface.

12. A cooling head according to any of claims 1 to 11, further including a light within the chamber to illuminate the treatment area.

## Patentansprüche

1. Kühlkopf (1) für einen Hautbehandlungslaser, wobei der Kühlkopf aufweist:
einen Körper (2) mit
einer Montage (3) an einem Ende zur Anbringung an einem Laserkopf, um den Körper relativ zu dem Kopf in einer fixierten Position zu befestigen;
eine Kühlfläche (4) an dem anderen Ende zur Aufbringung auf der Haut eines Patienten;
eine Kühlkammer (5), die zwischen der Montage und der Kühlfläche angeordnet ist, wobei die Kammer hat:
Wandabschnitte (60, 61), die gegenüber CO₂-, Erbium-YAG- oder anderem, nicht sichtbarem Laserlicht transparent sind, um es einem Laserstrahl zu ermöglichen, von der Montage zu der Haut des Patienten über die Kühlfläche geführt zu werden und
einen Kühlmitteleinlaß (9) und -auslaß (8), die in dem Körper vorgesehen sind, um eine Zufuhr an Kühlmittel zu und die Entfernung von Kühlmittel von dem Körper bei Verwendung zu ermöglichen, um die Kühlfläche zu kühlen;
einen Ausführdurchgang (42) zum Ermöglichen der Entfernung von Überbleibseln aus dem Behandlungsbereich und zur Verbindung mit einer Vakuumquelle; und
ein Fenster (100) in dem Körper, das im Bereich sichtbaren Lichts optisch transparent ist, um es dem Laserstrahl bei der Kühlfläche zu ermöglichen, von einem den Laser verwendenden Chirurgen durch den Körper gesehen zu werden.

2. Kühlkopf nach Anspruch 1, wobei die transparenten Wandabschnitte ein Paar ausgerichteter bzw. fluchtender Fenster an gegenüberliegenden Seiten der Kühlmittelkammer aufweisen, wobei eines an die Montage angrenzt und das andere in der Kühlfläche ist oder an diese angrenzt.

3. Kühlkopf nach Anspruch 1 oder Anspruch 2, wobei der Ausführdurchgang mit einer Öffnung in der Kühlfläche verbunden ist, die an dem Wandabschnitt in der Kühlfläche angrenzt, durch den das Laserlicht auf der Haut des Patienten auftritt.

4. Kühlkopf nach Anspruch 1 oder Anspruch 2, wobei ein flexibler Schutz um die Kühlfläche vorgesehen ist, um eine Ausführkammer variablen Volumens auszubilden und woran der Ausführdurchgang, zusammen mit einem Lufteinlaßdurchgang anschließbar sind zum Zwecke der Entfernung von Überbleibseln.

5. Kühlkopf nach einem der Ansprüche 1 bis 4, wobei der gegenüber Laserlicht transparente Wandabschnitt in der Kühlfläche im sichtbaren Spektrum optisch transparent ist, um ein Betrachten der Hautoberfläche durch den Chirurgen zu ermöglichen.

6. Kühlkopf nach einem der Ansprüche 1 bis 4, wobei ein separates, transparentes Fenster in der Kühlfläche vorgesehen sein kann, um ein Betrachten der Hautoberfläche durch den Chirurgen zu ermöglichen.

7. Kühlkopf nach einem der Ansprüche 1 bis 6, wobei ein gegenüber sichtbarem Licht transparentes Fenster in einer angewinkelten Seitenwand des Körpers angeordnet ist, die in Richtung der Öffnung in der Kühlfläche weist.

8. Kühlkopf nach einem der Ansprüche 5 bis 7, wobei eines oder mehrere der transparenten Fenster die Form einer Linse hat, um den Behandlungsbereich zu vergrößern.

9. Kühlkopf nach einem der Ansprüche 1 bis 8, wobei zumindest die Kühlfläche des Körpers aus Metall ausgebildet ist, um der Fläche zu ermöglichen, daß sie wirkungsvoll durch den Strom an Kühlmittel durch die Kühlmittelkammer in dem Körper gekühlt wird, um eine Kühlung der Haut des Patienten durch Aufbringung der gekühlten Fläche auf der Haut, vorzusehen.

10. Kühlkopf nach einem der Ansprüche 1 bis 9, wobei die Montage einen rinförmigen Senker an dem offenen Ende der Bohrung umfaßt und Befestigungen zur Befestigung eines Laserkopfes in einer definierten Position innerhalb des Senkers hat.

11. Kühlkopf nach einem der Ansprüche 1 bis 10, wobei der Kühlmitteldurchgang Einlaß- und Auslaßdurchgänge aufweist, sowie eine innere Kammer, die innerhalb des Körpers ausgebildet ist und sich über die Fläche zu der Haut des Patienten hin öffnet.

12. Kühlkopf nach einem der Ansprüche 1 bis 11, des weiteren mit einem Licht innerhalb der Kammer zur Beleuchtung des Behandlungsbereichs.

## Revendications

1. Tête de refroidissement (1) pour laser de traitement de la peau, la tête de refroidissement comprenant :
un corps (2) comportant
un support (3) en une extrémité pour la fixation à une tête de laser pour maintenir le corps par rapport à la tête dans une position fixée,
une surface de refroidissement (4) à l'autre extrémité destinée à être appliquée à la peau d'un patient,
une chambre à fluide de refroidissement (5) disposée entre le support et la surface de refroidissement, la chambre comportant
des parties de paroi (60, 61) transparentes à la lumière de laser CO2, de laser Erbium-YAG ou autre lumière de laser non visible pour permettre à un faisceau laser de passer du support à la peau du patient à travers la surface de refroidissement, et
un orifice d'entrée (9) et un orifice de sortie (8) de fluide de refroidissement prévus dans le corps pour permettre l'alimentation et le retrait de fluide de refroidissement dans le corps en utilisation afin de refroidir la surface de refroidissement,
un orifice d'extraction (42) permettant le retrait de débris de la zone de traitement et pour la connexion à une source de vide, et
une fenêtre (100) dans le corps optiquement transparente dans la plage de lumière visible pour permettre à un chirurgien utilisant le laser de voir le faisceau laser au niveau de la surface de refroidissement à travers le corps.

2. Tête de refroidissement selon la revendication 1, dans laquelle les parties de paroi transparentes comprennent une paire de fenêtres alignées sur des côtés opposés de la chambre à fluide de refroidissement, l'une adjacente au support et l'autre dans ou adjacente à la surface de refroidissement.

3. Tête de refroidissement selon la revendication 1 ou 2, dans laquelle l'orifice d'extraction est connecté à une ouverture dans la surface de refroidissement adjacente à la partie de paroi de la surface de refroidissement à travers laquelle la lumière du laser arrive sur la peau du patient.

4. Tête de refroidissement selon la revendication 1 ou 2, dans laquelle une jupe souple est placée autour de la surface de refroidissement pour former une chambre d'extraction de volume variable et à laquelle on peut connecter l'orifice d'extraction, ainsi qu'un orifice d'entrée d'air, pour permettre le retrait des débris.

5. Tête de refroidissement selon l'une quelconque des revendications 1 à 4, dans laquelle la partie de paroi transparente à la lumière laser de la surface de refroidissement est optiquement transparente dans le spectre visible pour permettre au chirurgien de voir la surface de la peau.

6. Tête de refroidissement selon l'une quelconque des revendications 1 à 4, dans laquelle une fenêtre transparente distincte peut être prévue dans la surface de refroidissement pour permettre au chirurgien de voir la surface de la peau.

7. Tête de refroidissement selon l'une quelconque des revendications 1 à 6, dans laquelle une fenêtre transparente à la lumière visible est placée dans une paroi latérale inclinée du corps tournée vers l'ouverture de la surface de refroidissement.

8. Tête de refroidissement selon l'une quelconque des revendications 5 à 7, dans laquelle une ou plusieurs des fenêtres transparentes se présente sous forme d'une lentille pour agrandir la zone de traitement.

9. Tête de refroidissement selon l'une quelconque des revendications 1 à 8, dans laquelle au moins la surface de refroidissement du corps est en métal afin de permettre à la surface d'être refroidie efficacement par l'écoulement de fluide de refroidissement à travers la chambre à fluide de refroidissement dans le corps afin de réaliser le refroidissement de la peau du patient par application de la surface refroidie sur la peau.

10. Tête de refroidissement selon l'une quelconque des revendications 1 à 9, dans laquelle le support comprend un épaulement interne annulaire à l'extrémité ouverte de l'alésage et comporte des fixations pour attacher une tête de laser dans une position définic à l'intérieur de l'épaulement interne.

11. Tête de refroidissement selon l'une quelconque des revendications 1 à 10, dans laquelle le passage de fluide de refroidissement comprend des orifices d'entrée et de sortie et une chambre interne formée dans le corps et débouchant à la peau du patient à travers la surface.

12. Tête de refroidissement selon l'une quelconque des revendications 1 à 11, comprenant en outre une lampe à l'intérieur de la chambre pour éclairer la zone de traitement.
